# FASCICULE DE BREVET EUROPEEN

(11) **EP 4 329 680 B1**
(45) Date de publication et mention de la délivrance du brevet: **30.10.2024**
(21) Numéro de dépôt: 22724105.6
(22) Date de dépôt: 22.04.2022
(51) Int. Cl.: A61F 2/38, A61B 5/00, A61F 2/46

(54) **DISPOSITIF MÉDICAL DE MESURE**
MEDIZINISCHE MESSVORRICHTUNG
MEDICAL MEASURING DEVICE

(30) Priorité: 27.04.2021 FR 2104368
(43) Date de publication de la demande: 06.03.2024
(73) Titulaire: Commissariat à l'Energie Atomique et aux Energies Alternatives, 75015 Paris (FR)
(72) Inventeur: GASNIER, Pierre, 38054 Grenoble cedex 09 (FR); BRULAIS, Sébastien, 38054 Grenoble cedex 09 (FR); NONGLATON, Guillaume, 38054 Grenoble cedex 09 (FR); RAMMOUZ, Ramzy, 38054 Grenoble cedex 09 (FR)
(74) Mandataire: INNOV-GROUP
(86) Numéro de dépôt international: PCT/EP2022/060692
(87) Numéro de publication internationale: WO 2022/229016

(56) Documents cités:
- WO-A2-2013/044160
- US-A1- 2003 069 644
- US-A1- 2005 113 932
- US-A1- 2007 234 819

## Description

### Domaine technique de l'invention

La présente invention se rapporte à un dispositif médical de mesure destiné à être placé dans le corps d'un être vivant et à un composant d'une prothèse incluant ledit dispositif médical de mesure.

### Etat de la technique

De manière connue, un implant de type prothèse de genou se compose d'un composant fémoral destiné à être fixé au fémur et d'un composant tibial destiné à être fixé au tibia. Un insert en polymère peut être employé comme interface d'appui entre le composant fémoral et le composant tibial.

Pour mieux comprendre les contraintes mécaniques qui s'exercent sur la prothèse, il est connu d'ajouter des capteurs dans le dispositif formant la prothèse. Ces capteurs sont généralement des jauges de contraintes intégrées au composant tibial et destinées à mesurer l'intensité et la direction des contraintes mécaniques qui s'exercent sur le composant. Une unité électronique est chargée de collecter les mesures et de les transmettre vers l'extérieur, via une liaison sans-fil.

Plusieurs documents antérieurs décrivent de telles solutions. Parmi eux, on peut citer les demandes de brevets EP2011455A1, WO2015/196131A1, ainsi que les brevets US8979758B2, US7097662B2 et US6821299B2.

Comme décrit dans le brevet US6821299B2, le composant tibial peut se composer d'une enveloppe externe dans laquelle vient se loger un insert portant les jauges de contraintes. Dans la demande de brevet WO2015/196131A1 le composant tibial comporte une partie portant une quille venant se loger dans le tibia du patient et présentant un plateau fixe de support et un plateau dit d'épreuve contre lequel vient s'appuyer le composant fémoral. A l'interface entre le plateau fixe et le plateau d'épreuve, le composant intègre une unité de mesure des forces venant s'exercer sur le plateau d'épreuve et une unité électronique destinée à collecter les mesures, à envoyer les mesures collectées et à alimenter l'ensemble des composants.

Les demandes de brevet US2005/113932A1 et US2003/069644A1 décrivent une prothèse tibia-fémur.

Les solutions antérieures, notamment au niveau du composant tibial, sont souvent composées de plusieurs pièces assemblées entre elles, ce qui présente certains inconvénients :
- Solidité et robustesse du composant au cours du temps ;
- Gestion de l'étanchéité entre les pièces ;

Par ailleurs, il n'est pas toujours aisé de positionner les différents capteurs, car les surfaces de réception ne sont pas toujours planes, ni facilement accessibles.

Le but de l'invention est de proposer un dispositif médical de mesure destiné à être employé pour mesurer les contraintes mécaniques présentes dans une articulation, ce dispositif étant particulièrement robuste au cours du temps et présentant une structure peu soumise à des contraintes d'étanchéité. Par conception, il facilite le report des jauges de contraintes et donc les coûts de fabrication d'un tel dispositif.

### Exposé de l'invention

Ce but est atteint par un dispositif médical de mesure destiné à être placé dans le corps d'un être vivant, pour mesurer les contraintes mécaniques d'une articulation présente entre deux parties du squelette de l'être vivant, comportant :
- un corps présentant un plateau fixe, un plateau dit d'épreuve sur lequel sont appliqués les contraintes mécaniques et un pilier se dressant sur une première face dudit plateau fixe et supportant le plateau d'épreuve,
- au moins une unité de mesure comprenant un ou plusieurs capteurs, agencé sur une deuxième face dudit plateau fixe, à l'opposé de ladite première face,
- le plateau fixe comportant un rebord externe remontant en périphérie du plateau d'épreuve et espacé dudit plateau d'épreuve d'un interstice.

Selon une particularité, le dispositif comporte un pont assurant une liaison entre le plateau d'épreuve et ledit rebord, comblant et/ou recouvrant ledit interstice.

Selon une autre particularité, le corps du dispositif est réalisé sous la forme d'un élément monobloc.

Selon une autre particularité, le plateau fixe comporte sur sa première face un premier renfoncement au fond duquel est placé ledit pilier supportant ledit plateau d'épreuve.

Selon une autre particularité, le plateau fixe comporte sur sa deuxième face un deuxième renfoncement, dans lequel est logée ladite unité de mesure.

Selon une autre particularité, chaque capteur est une jauge de contrainte.

Selon une autre particularité, l'unité de mesure comporte au moins quatre jauges de contrainte formées par un pont de mesure de type Wheatstone.

Selon une autre particularité, l'unité de mesure comporte une pastille formant un unique support des jauges de contrainte, ladite pastille étant fixée sur ladite deuxième face du plateau fixe.

Selon une autre particularité, le corps est réalisé en titane ou dans un alliage chrome-cobalt.

Selon une autre particularité, le dispositif comporte au moins un organe de bridage du déplacement du plateau d'épreuve par rapport au plateau fixe.

L'invention concerne également un composant d'une prothèse destiné à être placé au niveau d'une articulation d'un être vivant, ledit composant comportant :
- un dispositif médical de mesure tel que défini ci-dessus,
- une quille comprenant au moins un embout fixé au corps du dispositif médical de mesure, son embout délimitant au moins un espace étanche dans lequel est placée ladite unité de mesure,
- une unité électronique logée dans ladite quille, à laquelle est connectée ladite unité de mesure.

Selon une particularité, ledit espace comporte un premier sous-espace dans lequel est placée ladite unité de mesure et un circuit de traitement de ladite unité électronique, et un deuxième sous-espace séparé dudit premier sous-espace, dans lequel est placée un circuit de communication et d'alimentation de ladite unité électronique.

Selon une autre particularité, le circuit de communication et d'alimentation fonctionne par technologie inductive.

### Brève description des figures

D'autres caractéristiques et avantages vont apparaître dans la description détaillée qui suit faite en regard des dessins annexés dans lesquels :
- Les figures 1A à 1D représentent le dispositif médical de mesure selon un premier mode de réalisation ;
- La figure 2 représente une variante de réalisation du dispositif des figures 1A à 1D ;
- Les figures 3A à 3C représentent le dispositif médical de mesure de l'invention, selon une variante de réalisation ;
- La figure 4 représente le dispositif médical de mesure de l'invention, vu en coupe longitudinale, selon une autre variante de réalisation ;
- La figure 5 représente le dispositif médical de mesure de l'invention, vu en coupe longitudinale, selon une autre variante de réalisation ;
- La figure 6 représente le dispositif médical de mesure de l'invention, vu en coupe longitudinale, selon une autre variante de réalisation ;
- La figure 7 représente le dispositif médical de mesure de l'invention, vu en coupe longitudinale, selon une autre variante de réalisation ;
- La figure 8 illustre le principe de fonctionnement d'un ensemble de quatre jauges de contrainte montées suivant un pont de Wheatstone ;
- La figure 9 montre un exemple de répartition des capteurs dans le dispositif de mesure ;
- La figure 10 représente, vu en perspective, le composant de l'invention intégrant le dispositif médical de mesure ;
- Les figures 11A et 11B représentent deux variantes de réalisation du composant de l'invention ;
- La figure 12 illustre le principe de calibration du dispositif médical de mesure de l'invention ;

### Description détaillée d'au moins un mode de réalisation

Le composant de l'invention est celui destiné à être utilisé dans une prothèse d'une articulation d'un être vivant.

Dans la suite de la description, les termes "au-dessus", "au-dessous", "supérieur" et "inférieur" sont à considérer en tenant compte d'un axe (Z) tracé verticalement et orienté vers le haut sur les dessins annexés.

Une prothèse d'une articulation telle qu'un genou comporte un composant tibial destiné à être fixé au tibia du patient et un composant fémoral destiné à être fixé au fémur du patient. A l'interface entre le composant fémoral et le composant tibial, la prothèse peut comporter un insert en matériau polymère ou équivalent.

Le composant 1 de l'invention correspond au composant de la prothèse sur lequel sont exercées les contraintes mécaniques au niveau de l'articulation. Dans le cas d'une prothèse de genou, il s'agit avantageusement du composant tibial.

Avantageusement, le composant 1 de l'invention se compose uniquement de deux pièces assemblées entre elles, un dispositif 2 médical de mesure destiné à la mesure et un ensemble fixé audit dispositif 2 et portant l'électronique de traitement des mesures et de communication des mesures effectuées.

Le composant 1 et son dispositif 2 médical de mesure peuvent être adaptés pour être employés dans une prothèse de hanche, de genou, d'épaule ou de toute autre articulation.

Selon un aspect particulier de l'invention, le dispositif 2 médical de mesure de l'invention peut être employé dans le composant 1 selon différentes configurations.

Selon une première configuration, il peut être installé à demeure dans le corps de l'être vivant, et intégré au composant de la prothèse, par exemple le composant tibial 1. Il est alors autonome dans son fonctionnement.

Selon une deuxième configuration, il peut être utilisé comme insert temporaire entre deux composants de l'articulation, venant par exemple s'adapter temporairement sur le composant tibial. Il peut ainsi être remplaçable et adaptable à la morphologie de l'articulation à observer. Dans cette configuration temporaire, il peut être inséré pendant l'opération chirurgicale de l'articulation et employé pour mesurer les contraintes mécaniques en vue de choisir ultérieurement le composant adapté au patient.

Dans les deux configurations, il peut par exemple être alimenté électriquement par l'extérieur, par exemple via une liaison USB ou équivalent, ou via une transmission d'énergie sans contact (par exemple par couplage inductif).

La description du dispositif ci-dessous est valable pour les deux configurations.

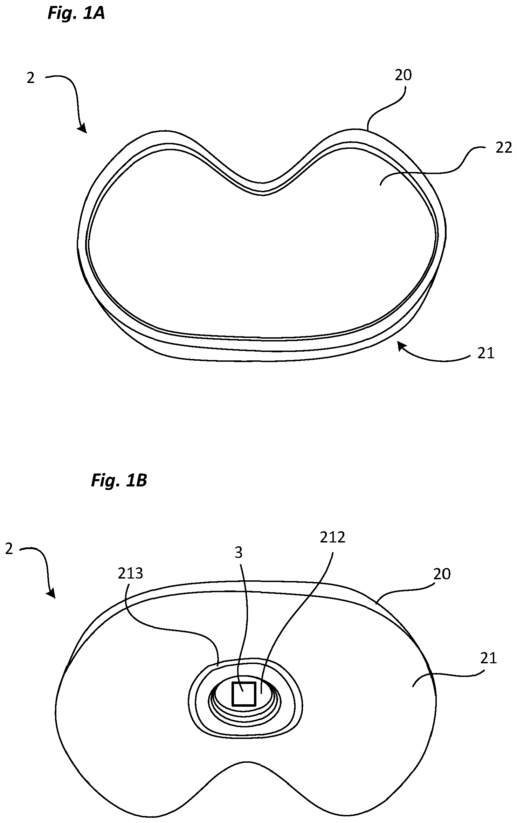

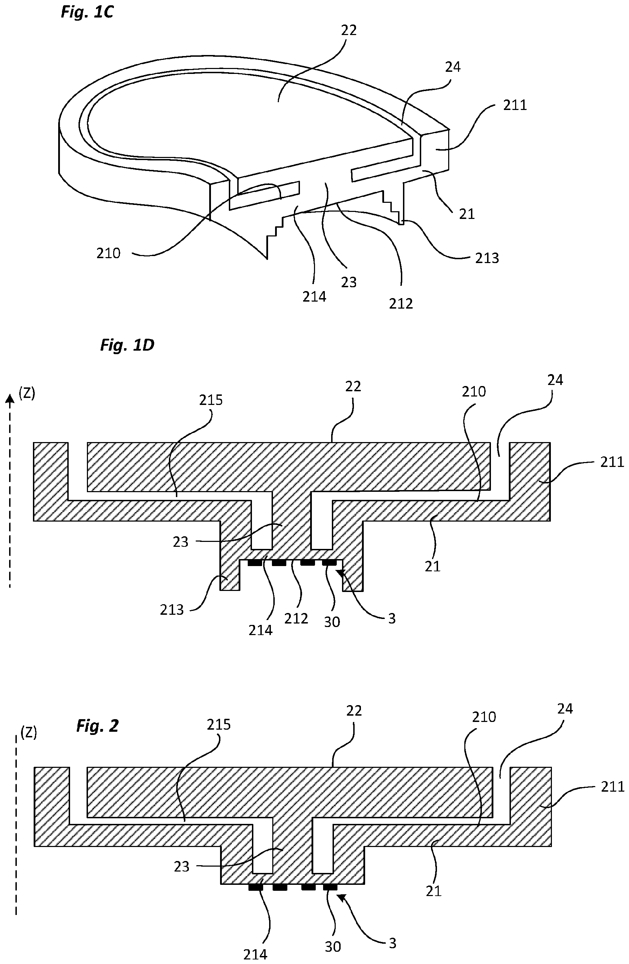

En référence à la figure 1A, à la figure 1B, figure 1C et à la figure 1D, le dispositif 2 médical de mesure comporte un corps 20.

En référence à ces figures, le corps 20 comporte un plateau fixe 21, un plateau dit d'épreuve 22 car supportant les contraintes mécaniques, et un seul pilier 23 se dressant sur une première face dudit plateau fixe 21 et supportant le plateau d'épreuve 22. De manière non limitative, pour une articulation de type genou, le plateau fixe 21 peut présenter une section de forme adaptée au type d'articulation à traiter.

De manière non limitative, le plateau d'épreuve 22 s'étend au-dessus du plateau fixe 21, séparé de ce dernier par le pilier 23. L'espace 215 présent entre le plateau fixe 21 et le plateau d'épreuve 22 définit le niveau de déplacement possible du plateau d'épreuve 22 par rapport au plateau fixe 21. Cet espace est défini par la forme du plateau d'épreuve 22 sur sa face inférieure reliée au pilier, et par la forme du plateau fixe 21 sur sa face supérieure, sur laquelle se dresse le pilier 23. Les deux faces peuvent être planes et parallèles entre elles. Dans une autre configuration non représentée, la face inférieure du plateau d'épreuve 22 peut par exemple présenter des flans évasés.

Comme représenté sur la figure 1C, le plateau fixe 21 peut présenter un premier renfoncement 210 sur sa première face présentant un fond, par exemple plan, sur lequel se dresse le pilier 23 supportant le plateau d'épreuve 22, ledit renfoncement 210 étant délimité par un bord 211 externe donnant sa forme audit plateau fixe 21 et remontant pour protéger latéralement ledit plateau d'épreuve 22. Un interstice 24 périphérique est ainsi créé entre le bord externe du plateau d'épreuve 22 et le rebord 211 du plateau fixe.

Sur sa deuxième face, opposée à sa première face, le plateau fixe 21 porte une unité de mesure 3, composée d'un ou plusieurs capteurs. Cette unité de mesure 3 est destinée à mesurer les contraintes mécaniques qui viennent s'exercer sur le plateau d'épreuve 22. Dans le cas d'une prothèse de genou, ces contraintes mécaniques sont exercées par le composant fémoral, venant en appui sur le plateau d'épreuve, éventuellement à travers l'insert situé à l'interface.

De manière non limitative, comme représenté sur les figures 1A à 1D, l'unité de mesure 3 peut être logée dans un deuxième renfoncement 212 axial ménagé sur la deuxième face, sous le pilier 23, ce deuxième renfoncement 212 étant délimité par un bord 213 par exemple circulaire. Ce deuxième renfoncement 212 forme ainsi un logement dans lequel est placée l'unité de mesure 3. Le fond de ce deuxième renfoncement 212 est formé d'une surface avantageusement plane. Les jauges de contrainte 30 sont par exemple collées sur cette surface. La figure 2 montre une variante de réalisation du dispositif, dénuée de ce deuxième renfoncement 212. D'autres architectures pourraient bien entendu être envisagées.

Selon un aspect particulier de l'invention, il est possible de créer une liaison entre le plateau fixe 21 et le plateau d'épreuve, en formant un pont 240, 241, 242 entre le plateau d'épreuve 22 et le rebord 211 du plateau fixe 21. La face supérieure du dispositif est ainsi une face pleine, ledit pont s'étendant latéralement pour venir combler et/ou recouvrir l'interstice 24 présent entre le plateau d'épreuve 24 et le rebord 211.

Cette solution permet notamment de :
- Eviter le développement de bactéries (aucun interstice/cavités) ;
- Limiter le déplacement du plateau d'épreuve 22 et donc les contraintes mécaniques sur le pilier 23 et la membrane (214, voir ci-après) ;

Il faut noter que la liaison est réalisée avec une épaisseur adaptée permettant une déformation mesurable au niveau de l'unité de mesure 3, via le pilier 23 qui joint le plateau d'épreuve 22 au plateau fixe 21, tout en respectant la limite d'élasticité du matériau utilisé pour la fabrication du corps 20 du dispositif (par exemple le titane) ainsi que la limite d'utilisation des capteurs.

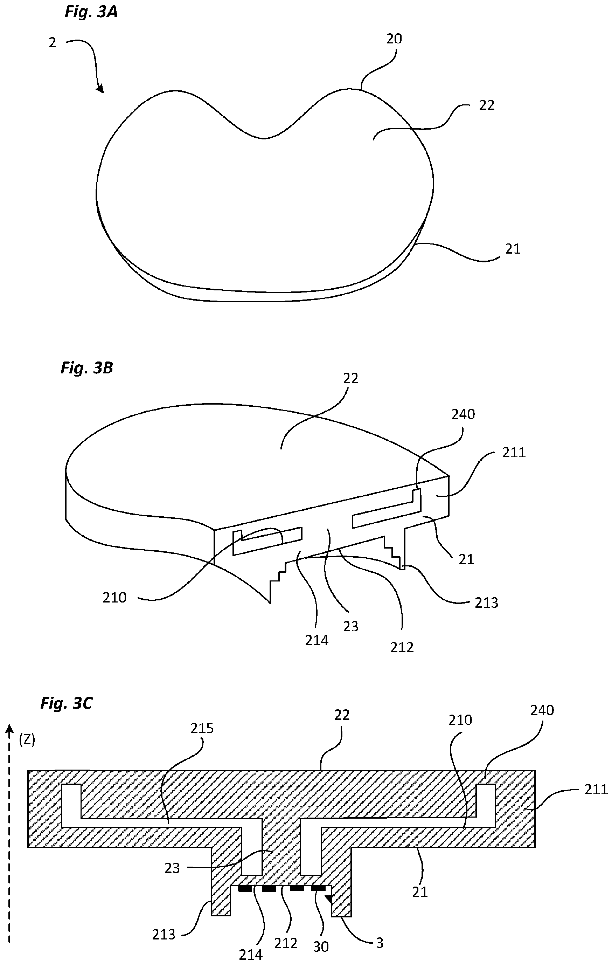

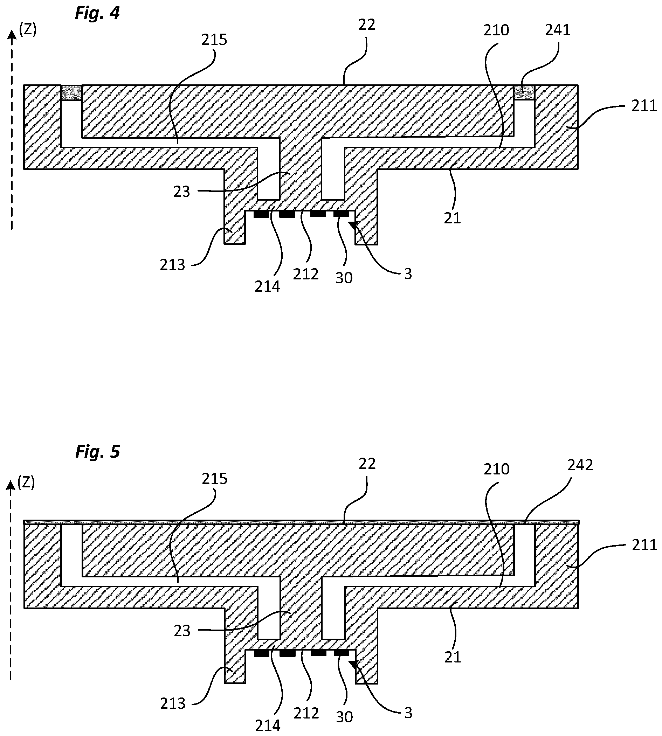

Dans une première variante de réalisation illustrée par la figure 3A, la figure 3B et la figure 3C, le pont 240 venant combler partiellement l'interstice 24 périphérique présent entre le plateau d'épreuve 22 et le rebord 211 du plateau fixe est réalisé en prolongeant le plateau d'épreuve 22 vers l'extérieur jusqu'à joindre le rebord 211 du plateau fixe 21. Dans une variante de réalisation représentée sur la figure 4, le pont 241 est formé par un matériau venant combler l'interstice 24 présent entre le rebord 211 et le plateau d'épreuve 22. Il peut notamment s'agir d'un matériau de colmatage, de type résine, avantageusement de consistance plus molle que celle du reste du corps (par exemple en titane). Cette dernière solution permettrait de garantir un bridage mécanique du plateau d'épreuve 22 tout en limitant la pénétration d'impuretés ou de bactéries entre les deux plateaux.

Dans une autre variante de réalisation représentée sur la figure 5, le pont 242 est réalisé par une couche de matériau déposée sur le plateau d'épreuve 22 et sur la face supérieure du rebord 221, recouvrant l'interstice 24 présent entre le plateau d'épreuve 22 et le rebord 211.

Il faut noter qu'il est également possible de mixer plusieurs des modes de réalisation de cette liaison.

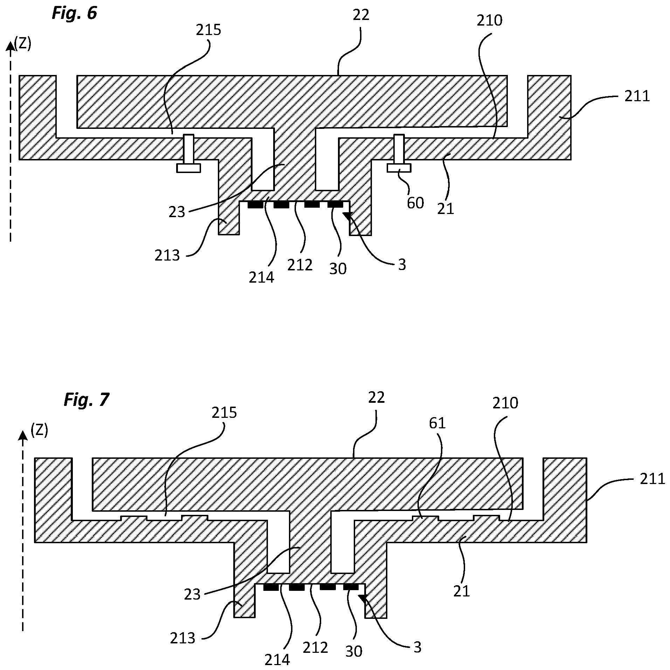

Selon une particularité de l'invention illustrée par la figure 6 et par la figure 7, le dispositif 1 peut comporter un dispositif de bridage du déplacement du plateau d'épreuve 22 par rapport au plateau fixe 21. Ce dispositif, représenté sur la figure 6 et la figure 7, peut comporter un ou plusieurs organes de butée venant se positionner sous le plateau d'épreuve 22, pour adapter le niveau de déplacement du plateau d'épreuve 22, lorsque celui-ci est soumis à des contraintes, en jouant sur l'espace 215 de déplacement du plateau d'épreuve 22 par rapport au plateau fixe 21. Dans une première réalisation représentée sur la figure 6, deux organes de butée sont réalisés sous la forme d'une ou plusieurs vis 60 insérées à travers le plateau fixe 21, par le dessous, et dont les extrémités débouchent sous le plateau d'épreuve 22. L'extrémité de chaque vis forme la butée réglable.

Dans une deuxième réalisation représentée sur la figure 7, chaque organe de butée est formé d'un plot 61 sur le plateau fixe 21, se dressant dans l'espace 215 existant entre le plateau fixe 21 et le plateau d'épreuve 22. Les plots 61 sont avantageusement répartis de manière équilibrée autour du pilier 23.

De manière avantageuse, le corps 20 du dispositif 2 de mesure se présente sous la forme d'un élément entièrement monobloc. Le corps 20 peut être réalisé dans des matériaux métalliques ou en céramique. De manière avantageuse, il peut être fabriqué en titane ou dans un alliage de type chrome-cobalt. Il peut être obtenu par fabrication additive. Dans cette configuration, le plateau fixe 21, le pilier et le plateau d'épreuve sont réalisés en une seule pièce. La réalisation des figures 3A à 3C rentre aussi dans ce cadre, le pont 240 réalisant la liaison entre le rebord 211 et le plateau d'épreuve 22 faisant partie de l'ensemble monobloc. La fabrication additive reste applicable à cette réalisation. Dans la réalisation de la figure 4 et celle de la figure 5, le pont 241, 242 peut être obtenu en réalisant un dépôt supplémentaire sur le corps 20 monobloc du dispositif.

Selon une particularité, chaque capteur se compose par exemple d'une jauge de contrainte 30.

De manière non limitative, l'unité de mesure 3 comporte plusieurs jauges de contraintes 30 et ces jauges de contrainte 30 peuvent être montés suivant deux ponts de type Wheatstone. Chaque pont de Wheatstone comporte ainsi quatre jauges de contrainte. Les jauges de contrainte exploitent l'effet piézo-résistif des métaux (résistivité variant avec la déformation). Elles sont positionnées de manière optimisée, pour permettre de maximiser leur déformation longitudinale et donc la sensibilité en couple et en force du capteur.

Une jauge de contrainte 30 de ce type est basée sur la propriété qu'ont certains matériaux de voir leur conductivité varier lorsqu'ils sont soumis à des contraintes, pressions ou déformations. Alimenté par une source de tension, le pont de Wheatstone présente, à l'équilibre, une tension nulle, mais la variation de l'une ou l'autre des résistances, du fait de la déformation, fait apparaître une tension non nulle.

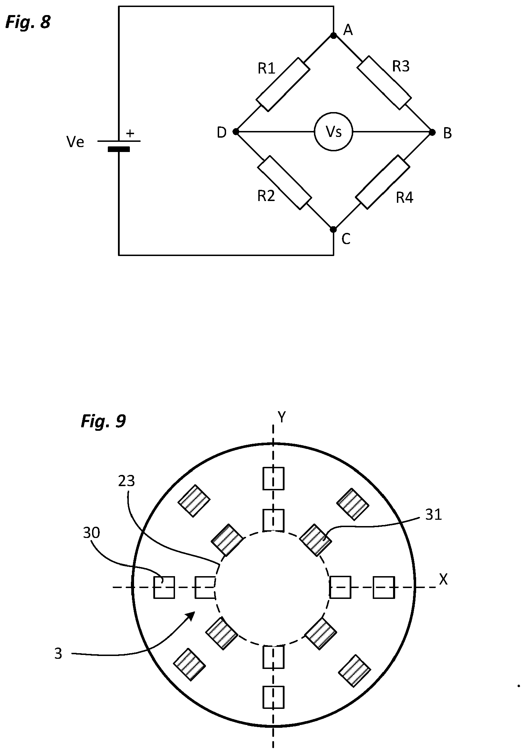

Le principe de fonctionnement de quatre jauges de contrainte connectées sous la forme d'un pont de Wheatstone est décrit ci-dessous en liaison avec la figure 8.

Soit un circuit constitué de quatre résistances R1, R2, R3, R4 montées en pont suivant quatre bornes A, B, C, D. On alimente par une source électromotrice Ve suivant les deux bornes A, C. À l'équilibre la tension de sortie entre les bornes B et D est nulle mais la variation d'une quelconque des résistances fait apparaître une tension de sortie Vs entre les deux bornes B et D. La mesure de cette tension permet de déduire la force ou le couple imposée par l'utilisateur sur le plateau d'épreuve 22.

La deuxième face du plateau fixe 21 est avantageusement plane pour venir facilement y fixer l'unité de mesure 3 comprenant lesdites jauges de contraintes 30.

A titre d'exemple, comme représenté sur la figure 9, des premières jauges de contrainte sont au nombre de huit, divisées en une première série de quatre jauges de contrainte 3 alignées suivant un premier axe (axe X), formant le premier pont de Wheatstone, et une deuxième série de quatre jauges alignées suivant un deuxième axe, perpendiculaire au premier axe (axe Y), formant le deuxième pont de Wheatstone. Au niveau de l'emplacement des jauges 30, sous le pilier 23, le plateau fixe 21 dispose ainsi d'une membrane 214 déformable mécaniquement selon la force venant s'exercer sur le plateau d'épreuve 22. Cette membrane 214 peut présenter une forme de disque ou toute autre forme (par exemple ovale ou autres), au centre duquel est placé le pilier 23. Ces premières jauges de contrainte 30 permettent de mesurer la force exercée sur le plateau d'épreuve 22 selon l'axe Z, ainsi que les moments des forces selon l'axe X et selon l'axe Y.

L'unité de mesure peut comporter avantageusement un deuxième jeu de par exemple huit jauges de contrainte 31, réparties en deux ponts de Wheatstone. Elles sont agencées pour mesurer le moment de la force selon l'axe Z. De manière non limitative, ces jauges de contrainte 31 du deuxième jeu sont organisées selon deux axes perpendiculaires, décalés angulairement par rapport aux jauges de contrainte 30 du premier jeu.

De manière non limitative, le dispositif 2 médical de mesure peut présenter les caractéristiques dimensionnelles suivantes :

| | |
|---|---|
| **Hauteur pilier** | 1 mm |
| **Diamètre pilier** | 6 mm |
| **Epaisseur membrane** | 2 mm |
| **Hauteur sous plateau d'épreuve** | 1 mm |
| **Diamètre membrane (bas)** | 16 mm |
| **Diamètre membrane (haut)** | 17 mm |
| **Epaisseur plateau d'épreuve** | 3 mm |

Il faut noter que les dimensions sont choisies pour permettre un compromis entre :
- Les contraintes maximales que subissent le pilier 23 et la membrane 214. A titre d'exemple, le pilier 23 a été dimensionné pour une force maximale de 2600N déportée au niveau d'un condyle unique du fémur.
- La place disponible selon les lignes X et Y sur la membrane 214 pour le report des jauges.

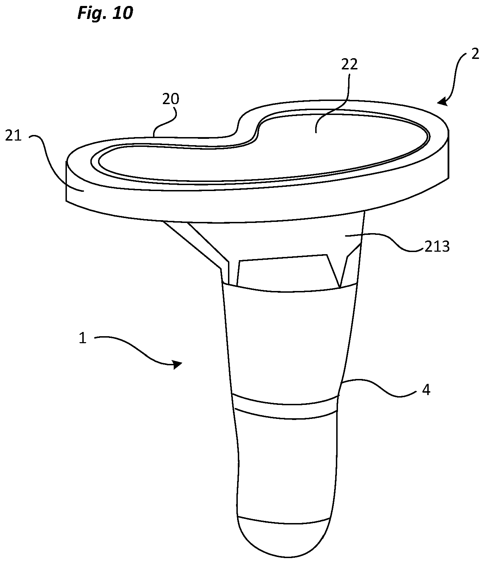

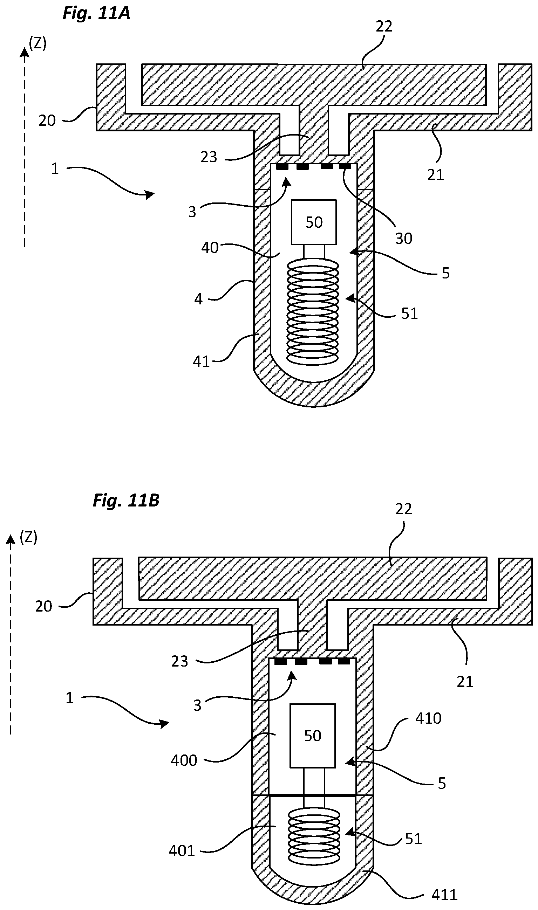

La figure 10 et la figure 11A et la figure 11B représentent des exemples de réalisation du composant intégrant le dispositif 2 médical de mesure. Les particularités décrites ci-dessus pour le dispositif sont toutes utilisables dans le composant.

En référence à la figure 10, pour isoler l'unité de mesure 3 par rapport à l'extérieur, le composant 1 comporte une quille 4 comprenant au moins un embout venant se fixer sur le corps 20 du dispositif 2, sur le bord 213 du deuxième renfoncement 212 et fermer au moins un espace dans lequel sont logées ladite unité de mesure 3 et une unité électronique 5 à laquelle est reliée l'unité de mesure 3. La quille 4 s'étend axialement à l'opposé du plateau d'épreuve 22.

L'unité électronique 5 peut comporter un circuit de traitement 50 et un circuit de communication et d'alimentation 51 comprenant notamment une antenne.

A titre d'exemple, l'embout peut être réalisé dans un matériau de type Polyuréthane, PEEK (poly-éther cétone) ou céramique.

En référence à la figure 7A et à la figure 7B, deux variantes de réalisation sont par exemple envisageables.

Sur la figure 11A, une première variante consiste à loger l'unité de mesure 3 et l'unité électronique 5 dans le même espace 40 délimitée par la quille, la quille 4 étant alors réalisée sous la forme d'une seule pièce 41 fixée sur le corps du dispositif.

Sur la figure 11B, l'espace peut être divisé en un premier sous-espace 400 dans lequel est logé le circuit de traitement 50 et l'unité de mesure 3 et un deuxième sous-espace 401 dans lequel est logé le circuit de communication et d'alimentation 51. Pour définir chaque sous-espace, la quille 4 peut comporter une première partie 410 venant se fixer sur le corps 20 du dispositif et enfermant de manière hermétique l'unité de mesure 3 et le circuit de traitement 50 de l'unité électronique et une deuxième partie 411, prolongeant la première partie et logeant le circuit de communication et d'alimentation 51. Dans cette variante, la première partie de l'embout peut être réalisée dans un matériau de type titane et la deuxième partie dans un matériau de type de Polyuréthane, PEEK (poly-éther cétone) ou céramique.

Une résine peut être injectée dans chaque espace 40 ou sous-espace 400, 401 afin de renforcer l'étanchéité du composant 1 autour de l'unité de mesure 3 et de l'unité électronique 5 et de maintenir mécaniquement les circuits électroniques, notamment le circuit de traitement 50.

Le circuit de communication et d'alimentation 51 peut comporter une antenne couplée à un circuit électronique de type RFID ou équivalent, permettant une communication et une alimentation électrique par technologie inductive.

La prise de mesure est commandée par un microcontrôleur du circuit de traitement 50 et les mesures effectuées sont transmises par le microcontrôleur vers l'extérieur via le circuit de communication et d'alimentation 51.

Le circuit de traitement 50 peut inclure un transistor commandable par le microcontrôleur, apte à mettre en conduction chaque jauge 30 de contrainte de manière totale, ou éventuellement partielle par demi branche de pont de Wheatstone ou pont de Wheatstone, uniquement lors de la conversion analogique numérique. La consommation est donc proportionnelle au rapport cyclique de mise en conduction des jauges.

Afin d'obtenir des résultats suffisamment probants, une procédure de calibration du dispositif 1 médical de mesure peut être prévue.

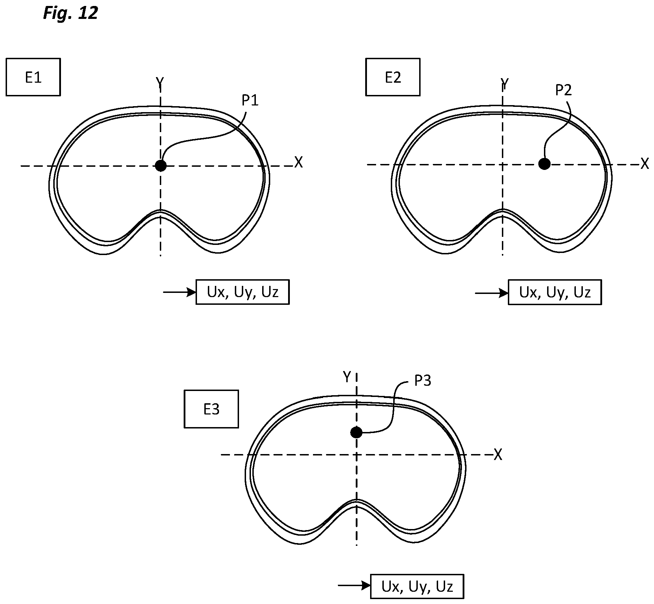

A titre d'exemple, en référence à la figure 12, la calibration peut être réalisée de la manière suivante :
∘ E1 : Réaliser un appui ponctuel statique P1 centré, c'est-à-dire en (0,0), sur le plan d'appui porté par le plateau d'épreuve 22 (le plateau d'épreuve pourra posséder un repère indiquant son centre exact) et mesurer les tensions *Uₓ, U_{y}* et *U_{z}.* Dans ce cas, les tensions *Uₓ, U_{y}* avoisinent normalement zéro. La sensibilité en force est alors calculée à partir de *U_{z}.* Pour plus de précision sur la sensibilité, plusieurs valeurs de forces peuvent être appliquées et leur valeur de U_{z} respectives mesurées ;
∘ E2 : Réaliser un appui ponctuel statique P2 décentré en x, par exemple en (x1,0) » avec x1 différent de 0, sur le plateau mobile et mesurer *Uₓ, U_{y}* et *U_{z}.* Dans ce cas, la tension *Uₓ* et *U_{z}* sont non nulles et *U_{y}* est censée être nulle. Connaissant la coordonnée d'application, la force appliquée selon z et la mesure de *U_{y},* la sensibilité en couple autour de l'axe y est alors calculée.
∘ E3 : Réaliser la même chose, avec un appui ponctuel statique P3 décentré en y, par exemple en (0,y1) avec y1 différent de 0, et mesurer.

L'invention présente de nombreux avantages parmi lesquels :
- Une solution particulièrement robuste, notamment en latéral, le plateau fixe 21 ayant son bord 211 qui remonte latéralement pour se positionner autour du plateau d'épreuve ; De cette manière, les contraintes latérales ne sont pas mesurées et ne viennent pas perturber les mesures réalisées selon l'axe Z.
- Le dispositif 2 de mesure peut être réalisé sous la forme d'un élément monobloc, ce qui permet d'éviter l'utilisation de colle, de moyens d'assemblage de type emboitement ou vissage. En outre, les contraintes d'herméticité au niveau du dispositif sont également évitées.
- Le pilier 23 forme l'unique liaison rigide entre le plateau fixe 21 et le plateau d'épreuve 22 et permet au plateau d'épreuve 22 de se déformer dans toutes les directions autour de l'axe du pilier 23.
- La création d'un pont entre le plateau d'épreuve 22 et le plateau fixe 21, permet d'éviter l'insertion de toute impureté/bactérie dans l'interstice créé entre les deux, sans restreindre le fonctionnement mécanique du dispositif.
- Le calibrage du dispositif 2 est aisé.
- Le report des huit jauges de contrainte 30 et des huit jauges de contrainte 31 sur une surface facilement accessible.
- Possibilité de personnaliser la prothèse à la corpulence du patient équipé, tout en conservant certains composants standards. Ceci est notamment permis par le fait que le pilier et la membrane sont situés au centre du plateau.

## Revendications

1. Dispositif (2) médical de mesure destiné à être placé dans le corps d'un être vivant, pour mesurer les contraintes mécaniques d'une articulation présente entre deux parties du squelette de l'être vivant, comportant :
- un corps (20) présentant un plateau fixe (21), un plateau dit d'épreuve (22) sur lequel sont appliqués les contraintes mécaniques et un pilier (23) se dressant sur une première face dudit plateau fixe (21) et supportant le plateau d'épreuve,
- au moins une unité de mesure (3) comprenant un ou plusieurs capteurs, agencé sur une deuxième face dudit plateau fixe, à l'opposé de ladite première face,
**caractérisé en ce que** :
- le plateau fixe (21) comporte un rebord (211) externe remontant en périphérie du plateau d'épreuve (22) et espacé dudit plateau d'épreuve d'un interstice (24).

2. Dispositif selon la revendication 1, **caractérisé en ce qu'**il comporte un pont (240, 241, 242) assurant une liaison entre le plateau d'épreuve (22) et ledit rebord (211), comblant et/ou recouvrant ledit interstice (24).

3. Dispositif selon la revendication 1 ou 2, **caractérisé en ce que** le corps (20) du dispositif est réalisé sous la forme d'un élément monobloc.

4. Dispositif selon l'une des revendications 1 à 3, **caractérisé en ce que** le plateau fixe (21) comporte sur sa première face un premier renfoncement (210) au fond duquel est placé ledit pilier (23) supportant ledit plateau d'épreuve (22).

5. Dispositif selon l'une des revendications 1 à 4, **caractérisé en ce que** le plateau fixe (21) comporte sur sa deuxième face un deuxième renfoncement (212), dans lequel est logée ladite unité de mesure (3).

6. Dispositif selon l'une des revendications 1 à 5, **caractérisé en ce que** chaque capteur est une jauge de contrainte (30).

7. Dispositif selon la revendication 6, **caractérisé en ce que** l'unité de mesure (3) comporte au moins quatre jauges de contrainte formées par un pont de mesure de type Wheatstone.

8. Dispositif selon la revendication 7, **caractérisé en ce que** l'unité de mesure comporte une pastille formant un unique support des jauges de contrainte, ladite pastille étant fixée sur ladite deuxième face du plateau fixe.

9. Dispositif selon l'une des revendications 1 à 8, **caractérisé en ce que** le corps (20) est réalisé en titane ou dans un alliage chrome-cobalt.

10. Dispositif selon l'une des revendications 1 à 9, **caractérisé en ce qu'**il comporte au moins un organe de bridage du déplacement du plateau d'épreuve (22) par rapport au plateau fixe (21).

11. Composant d'une prothèse destiné à être placé au niveau d'une articulation d'un être vivant, **caractérisé en ce qu'**il comporte :
- un dispositif (2) médical de mesure tel que défini dans l'une des revendications précédentes,
- une quille (4) comprenant au moins un embout fixé au corps (20) du dispositif (1) médical de mesure, son embout délimitant au moins un espace étanche dans lequel est placée ladite unité de mesure,
- une unité électronique (5) logée dans ladite quille (4), à laquelle est connectée ladite unité de mesure (3).

12. Composant selon la revendication 11, **caractérisé en ce que** ledit espace comporte un premier sous-espace (400) dans lequel est placée ladite unité de mesure (3) et un circuit de traitement (50) de ladite unité électronique (5), et un deuxième sous-espace (401) séparé dudit premier sous-espace, dans lequel est placée un circuit de communication et d'alimentation (51) de ladite unité électronique (5).

13. Composant selon la revendication 12, **caractérisé en ce que** le circuit de communication et d'alimentation (51) fonctionne par technologie inductive.

## Patentansprüche

1. Medizinische Vorrichtung (2) zum Messen, die dazu bestimmt ist, im Körper eines Lebewesens platziert zu werden, um die mechanischen Belastungen eines Gelenks zu messen, das sich zwischen zwei Teilen des Skeletts des Lebewesens befindet, umfassend:
- einen Körper (20), aufweisend eine feststehende Platte (21), eine Prüfplatte (22), auf welche die mechanischen Belastungen angewandt werden, und einen Pfeiler (23), der auf einer ersten Seite der feststehenden Platte (21) aufragt und die Prüfplatte stützt,
- mindestens eine einen oder mehrere Sensoren umfassende Messeinheit (3), die auf einer zweiten Seite der feststehenden Platte, entgegengesetzt zu der ersten Seite, angeordnet ist,
**dadurch gekennzeichnet, dass**:
- die feststehende Platte (21) einen äußeren Rand (211) aufweist, der am Umfang der Prüfplatte hochgezogen ist und von der Prüfplatte (22) um einen Zwischenraum (24) beabstandet ist.

2. Vorrichtung nach Anspruch 1, **dadurch gekennzeichnet, dass** sie eine Brücke (240, 241, 242) umfasst, die eine Verbindung zwischen der Prüfplatte (22) und dem Rand (211) gewährleistet und den Zwischenraum (24) ausfüllt und/oder überdeckt.

3. Vorrichtung nach Anspruch 1 oder 2, **dadurch gekennzeichnet, dass** der Körper (20) der Vorrichtung in Form eines einstückigen Teils ausgeführt ist.

4. Vorrichtung nach einem der Ansprüche 1 bis 3, **dadurch gekennzeichnet, dass** die feststehenden Platte (21) auf ihrer ersten Seite eine erste Vertiefung (210) umfasst, an deren Boden der Pfeiler (23) platziert ist, der die Prüfplatte (22) stützt.

5. Vorrichtung nach einem der Ansprüche 1 bis 4, **dadurch gekennzeichnet, dass** die feststehenden Platte (21) auf ihrer zweiten Seite eine zweite Vertiefung (212) umfasst, in der die Messeinheit (3) aufgenommen ist.

6. Vorrichtung nach einem der Ansprüche 1 bis 5, **dadurch gekennzeichnet, dass** jeder Sensor ein Belastungsmessstreifen (30) ist.

7. Vorrichtung nach Anspruch 6, **dadurch gekennzeichnet, dass** die Messeinheit (3) mindestens vier Belastungsmessstreifen umfasst, die durch eine Wheatstonesche Messbrücke gebildet werden.

8. Vorrichtung nach Anspruch 7, **dadurch gekennzeichnet, dass** die Messeinheit ein Plättchen umfasst, das eine einzige Stütze der Belastungsmessstreifen bildet, wobei das Plättchen auf der zweiten Seite der feststehenden Platte befestigt ist.

9. Vorrichtung nach einem der Ansprüche 1 bis 8, **dadurch gekennzeichnet, dass** der Körper (20) aus Titan oder aus einer Chrom-Cobalt-Legierung ausgeführt ist.

10. Vorrichtung nach einem der Ansprüche 1 bis 9, **dadurch gekennzeichnet, dass** sie mindestens ein Organ zur Drosselung der Bewegung der Prüfplatte (22) in Bezug auf die feststehende Platte (21) umfasst.

11. Komponente einer Prothese, die dazu bestimmt ist, an einem Gelenk eines Lebewesens platziert zu werden, **dadurch gekennzeichnet, dass** sie umfasst:
- eine medizinische Vorrichtung (2) zum Messen wie in einem der vorhergehenden Ansprüche definiert,
- einen Kegel (4), der mindestens ein Endstück umfasst, das an dem Körper (20) medizinischen Vorrichtung (1) zum Messen befestigt ist, wobei sein Endstück mindestens einen dichten Raum begrenzt, in dem die Messeinheit platziert ist,
- eine elektronische Einheit (5), die in dem Kegel (4) aufgenommen ist und an welche die Messeinheit (3) angeschlossen ist.

12. Komponente nach Anspruch 11, **dadurch gekennzeichnet, dass** der Raum einen ersten Teilraum (400) umfasst, in dem die Messeinheit (3) und eine Verarbeitungsschaltung (50) der Elektronikeinheit (5) platziert sind, und einen zweiten Teilraum (401), der von dem ersten Teilraum getrennt ist und in dem eine Kommunikations- und Versorgungsschaltung (51) der Elektronikeinheit (5) platziert ist.

13. Komponente nach Anspruch 12, **dadurch gekennzeichnet, dass** die Kommunikations- und Versorgungsschaltung (51) mit induktiver Technologie arbeitet.

## Claims

1. Medical measuring device (2) intended for placement in the body of a living being, for measuring the mechanical stresses of a joint connecting two parts of the skeleton of the living being, comprising:
- a body (20) having a fixed plate (21), a so-called proof plate (22) on which mechanical stresses are applied and a pillar (23) standing on a first face of said fixed plate (21) and supporting the proof plate,
- at least one measuring unit (3) comprising one or more sensors, arranged on a second face of said fixed plate, the opposite face from said first face,
**characterized in that**:
- the fixed plate (21) comprises an external rim (211) rising up at the periphery of the proof plate (22) and spaced from said proof plate by a gap (24).

2. Device according to Claim 1, **characterized in that** it comprises a bridge (240, 241, 242) providing a connection between the proof plate (22) and said rim (211), filling and/or covering the said gap (24).

3. Device according to Claim 1 or 2, **characterized in that** the body (20) of the device is produced as a one-piece element.

4. Device according to one of Claims 1 to 3, **characterized in that** the fixed plate (21) comprises on its first face a first recess (210) at the bottom of which said pillar (23) is placed supporting said proof plate (22).

5. Device according to one of Claims 1 to 4, **characterized in that** the fixed plate (21) comprises on its second face a second recess (212), in which said measuring unit (3) is housed.

6. Device according to one of Claims 1 to 5, **characterized in that** each sensor is a strain gauge (30).

7. Device according to Claim 6, **characterized in that** the measuring unit (3) comprises at least four strain gauges formed by a measuring bridge of the Wheatstone bridge type.

8. Device according to Claim 7, **characterized in that** the measuring unit comprises a pellet forming a single support for the strain gauges, said pellet being fixed to said second face of the fixed plate.

9. Device according to one of Claims 1 to 8, **characterized in that** the body (20) is made of titanium or of a chromium-cobalt alloy.

10. Device according to one of Claims 1 to 9, **characterized in that** it comprises at least one member for restraining the movement of the proof plate (22) with respect to the fixed plate (21).

11. Component of a prosthesis intended for placement at a joint of a living being, **characterized in that** it comprises:
- a medical measuring device (2) as defined in one of the preceding claims,
- a keel (4) comprising at least one endpiece fixed to the body (20) of the medical measuring device (1), its endpiece defining at least one sealed space in which said measuring unit is placed,
- an electronic unit (5) housed in said keel (4), to which said measuring unit (3) is connected.

12. Component according to Claim 11, **characterized in that** said space comprises a first sub-space (400) in which said measuring unit (3) and a processing circuit (50) of said electronic unit (5) are placed, and a second sub-space (401) separated from said first sub-space, in which a communication and power supply circuit (51) of said electronic unit (5) is placed.

13. Component according to Claim 12, **characterized in that** the communication and power supply circuit (51) operates by inductive technology.
